# EUROPÄISCHE PATENTSCHRIFT

(11) **EP 2 050 768 B1**
(45) Veröffentlichungstag und Bekanntmachung des Hinweises auf die Patenterteilung: **05.12.2012**
(21) Anmeldenummer: 08165609.2
(22) Anmeldetag: 01.10.2008
(51) Int. Cl.: C08F 4/80, B01J 23/42, C08F 4/70, C08F 30/08, C08L 43/04

(54) **Härtbare Siliconzusammensetzungen**
Curable silicone compositions
Composition silicone réticulable

(30) Priorität: 02.10.2007 DE 102007047212
(43) Veröffentlichungstag der Anmeldung: 22.04.2009
(73) Patentinhaber: Wacker Chemie AG, 81737 München (DE)
(72) Erfinder: Köllnberger, Dr. Andreas, 94431, Pilsting (DE)
(74) Vertreter: Fritz, Helmut

(56) Entgegenhaltungen:
- EP-A- 0 982 370
- EP-B- 0 948 565
- US-A- 3 188 300
- US-A- 4 108 833
- US-A- 4 157 426
- US-A- 4 329 275
- US-B1- 6 706 840

## Beschreibung

Die vorliegende Erfindung betrifft thermisch durch Hydrosilylierung vernetzbare Siliconzusammensetzungen, Verfahren zu deren Herstellung, hierzu eingesetzte Platinkatalysatoren sowie die Verwendung der vernetzbaren Zusammensetzungen.

Um additionsvernetzende Siliconzusammensetzungen durch die Hydrosilylierungsreaktion zu vernetzen wird im Allgemeinen ein Katalysator eingesetzt, der typischerweise Platin oder ein Metall aus der Platingruppe enthält. Bei der katalytisch ablaufenden Reaktion werden aliphatisch ungesättigte Gruppen mit Si-gebundenem Wasserstoff umgesetzt, um Netzwerkstrukturen auszubilden.

Bei zweikomponentigen Systemen werden die reaktiven Bestandteile erst kurz vor der Verarbeitung vermischt. Die Mischungen enthalten einen aktiven Platinkatalysator, was zur Folge hat, dass die Vernetzungsreaktion schon bei Raumtemperatur abläuft und die Zeit bis zur Verarbeitung (Topfzeit) eng begrenzt ist. Daraus ergeben sich Nachteile wie ein zusätzlicher Mischungsschritt, ein erhöhter Reinigungsaufwand bei technischen Störungen und die Gefahr von Platinkontaminationen in Behältern.

Der Bedarf an einkomponentigen additionsvernetzenden Siliconkautschuksystemen, die idealerweise bei Raumtemperatur überhaupt nicht und bei erhöhter Temperatur möglichst schnell aushärten, ist seit langer Zeit gegeben.

Es gibt verschiedene Ansätze, das Problem der vorzeitigen Vernetzung bei Raumtemperatur zu lösen. Eine Möglichkeit besteht in der Verwendung von Inhibitoren, die der Mischung als Additive zugesetzt werden, um eine Verlängerung der Topfzeit zu bewirken. Sie werden immer im Molaren Überschuss zur Katalysatorkomponente eingesetzt und hemmen dessen katalytische Aktivität. Mit zunehmender Inhibitormenge erfolgt jedoch neben einer Topfzeitverlängerung auch eine Abnahme der Reaktivität des Systems bei höheren Temperaturen und die Anspringtemperatur steigt an. Es gibt in der Literatur zahlreiche Beispiele für Inhibitoren aus verschiedenen Substanzklassen. In US 3,723,567 A werden aminofunktionelle Silane als Inhibitoren beansprucht. Alkyldiamine in Verbindung mit einem acetylenisch ungesättigten Alkohol werden in US 5,270,422 A zur Inhibierung verwendet. EP 0 761 759 A2 beansprucht eine Kombination von Inhibitoren, es wird ein Phosphit zusammen mit einem weiteren Inhibitor wie beispielsweise Maleate und Ethinole verwendet. DE 19 757 221 A1 beschreibt ebenfalls die Stoffklasse der Phosphite in der Verwendung eines Inhibitors. Phosphine werden in US 4,329,275 A als Additiv zur Inhibierung beansprucht. Eine Kombination von Phosphiten in Verbindung mit organischen Peroxyden wird von EP 1 437 382 A1 beschrieben. Neben negativen Einflüssen auf die Vernetzungskinetik erweist sich der Einsatz von zum Teil flüchtigen Inhibitoren oder Inhibitoren, die flüchtige Bestandteile freisetzen, ebenfalls als ungünstig. Mischungen, die eine völlige Inhibierung bei Raumtemperatur und die völlige Nichtbeeinflussung der Reaktionsgeschwindigkeit bei Aushärtebedingungen durch einen entsprechenden Zusatzstoff aufweisen, sind bisher nicht bekannt.

Eine weitere, davon grundsätzlich verschiedene, Möglichkeit besteht darin, den Katalysator in einem thermoplastischen Material zu verkapseln, welches bei erhöhter Temperatur schmilzt und dadurch den aktiven Katalysator freisetzt wie zum Beispiel in EP 0 459 464 A2 beschrieben. Die Herstellung des Katalysators ist jedoch relativ aufwändig.

Als dritte Möglichkeit, eine vorzeitige Vernetzung einkomponentiger Systeme bei Raumtemperatur zu verhindern ist die Verwendung spezieller Platinkomplexe. Platinalkinylkomplexe werden in US 6,252,028 B und in US 6,359,098 B beschrieben. In EP 0 982 370 A1 werden Platin-Alkin-Katalysatoren und deren Verwendung in härtbaren Silikonzusammensetzungen. In US 4,256,616 A kommen Pt(0)-phosphin und -phosphitkomplexe in Kombination mit Zinnsalzen zum Einsatz und WO 03/098 890 A1 beschreibt Pt(0)-phosphitkomplexe, die als Strukturmerkmale sowohl Phosphitliganden als auch Divinyldisiloxanliganden enthalten. US 6 706 840 B1 offenbart einen Triphenylphosphite-Platin-Komplex und deren Verwendung als Katalysator für Hydrosilylierungsreaktionen.

Obwohl die beschriebenen Massen deutlich verbesserte Topfzeiten bei zum Teil hinreichend hohen Vernetzungsgeschwindigkeiten bei einkomponentig formulierten, additionsvernetzenden Massen liefern, besteht weiterhin Bedarf an leistungsfähigeren Platinkatalysatoren, die bei erhöhter Temperatur eine schnelle Vernetzung des Materials gewährleisten, aber die oben genannte Nachteile nicht zeigen.

Aufgabe der vorliegenden Erfindung war es, additionsvernetzende Massen zur Verfügung zu stellen, die die oben genannten Nachteile nicht zeigen und neben verbesserten Tropfzeiten auch eine verbesserte Vernetzungsgeschwindigkeit ermöglichen.

Im Folgenden wird von dem Begriff Organopolysiloxane sowohl polymere, oligomere wie auch dimere Siloxane umfasst.

Gegenstand dieser Anmeldung sind additionsvernetzende Siliconzusammensetzungen, ausgewählt aus der Gruppe enthaltend
- jeweils mindestens eine Verbindung (A), (B) und (D),
- jeweils mindestens eine Verbindung (C) und (D), und
- jeweils mindestens eine Verbindung (A), (B), (C) und (D)
wobei
(A) eine organische Verbindung oder eine Siliziumorganische Verbindung, enthaltend mindestens zwei Reste mit aliphatischen Kohlenstoff-Kohlenstoff-Mehrfachbindungen,
(B) ein Siliziumorganische Verbindung, enthaltend mindestens zwei Si-gebundenen Wasserstoffatome,
(C) eine Siliziumorganische Verbindung, enthaltend SiC-gebundene Reste mit aliphatischen Kohlenstoff-Kohlenstoff-Mehrfachbindungen und Si- gebundene Wasserstoffatome, und
(D) ein Platinkatalysator bedeuten,
dadurch gekennzeichnet, dass der Platinkatalysator (D) der allgemeinen Formel (I) entspricht,

R¹₂Pt[P(C)R²)₃]₂ (I)

wobei
R¹ unabhängig voneinander, gleich oder verschieden, ein
   - Halogen,
   - einfach negativer anorganischer Rest,
   - lineare oder verzweigte aliphatische Reste der allgemeinen Formeln CᵥH₂ᵥ₊₁ oder CᵥH₂ᵥ₋₁ mit v = 1 bis 18 , Arylalkylreste mit 6 bis 31 Kohlenstoffatomen
   - -O-Alkyl, -O-Aryl, -O-Aryl-Alkyl,
   - -Si(Alkyl)₃, -Si(Aryl)₃, Si(Aryl-Alkyl)₃,
R² unabhängig voneinander, gleich oder verschieden, ein
   - Arylalkyl der Formel - (C₆H₅₋ₚ) - (CₒH₂ₒ₊₁)ₚ mit o = 1 - 31 und p = 1 - 5, wobei mindestens ein Alkylsubstituent an der 2-Position des Phenylrings gebunden ist,
   wobei die Wasserstoffatome der für R¹ und R² oben genannten Verbindungen substituiert oder nicht substituiert sind durch die Gruppen -NH₂, -COOH, -F, -Br, -Cl, -Aryl oder -Alkyl
bedeuten.

Es zeigte sich, dass die Platinkatalysatoren (D) insbesondere Pt(II)-phosphitkomplexe mit Platin in der Oxidationsstufe + II als Zentralmetall und Phosphor in der Oxidationsstufe + III zu den verbesserten Eigenschaften der erfindungsgemäßen Siliconzusammensetzungen führen.

Bei den erfindungsgemäßen Zusammensetzungen kann es sich um Einkomponenten-Siliconzusammensetzungen wie auch um Zweikomponenten-Siliconzusammensetzungen handeln. In letzterem Fall können die beiden Komponenten der erfindungsgemäßen Zusammensetzungen alle Bestandteile in beliebiger Kombination enthalten, im Allgemeinen mit der Maßgabe, dass eine Komponente nicht gleichzeitig Siloxane mit aliphatischer Mehrfachbindung, Siloxane mit Si-gebundenem Wasserstoff und Katalysator, also im Wesentlichen nicht gleichzeitig die Bestandteile (A), (B) und (D) bzw. (C) und (D) enthält. Vorzugsweise handelt es sich bei den erfindungsgemäßen Zusammensetzungen jedoch um Einkomponenten-Zusammensetzungen.

Die in den erfindungsgemäßen Zusammensetzungen eingesetzten Verbindungen (A) und (B) bzw. (C) werden bekanntermaßen so gewählt, dass eine Vernetzung möglich ist. So weist beispielsweise Verbindung (A) mindestens zwei aliphatisch ungesättigte Reste auf und (B) mindestens drei Si-gebundene Wasserstoffatome, oder Verbindung (A) weist mindestens drei aliphatisch ungesättigte Reste auf und Siloxan (B) mindestens zwei Si-gebundene Wasserstoffatome, oder aber anstelle von Verbindung (A) und (B) wird Siloxan (C) eingesetzt, welches aliphatisch ungesättigte Reste und Si-gebundene Wasserstoffatome in den oben genannten Verhältnissen aufweist. Auch sind Mischungen aus (A) und (B) und (C) mit den oben genannten Verhältnissen von aliphatisch ungesättigten Resten und Si-gebundenen Wasserstoffatomen.

Bei der erfindungsgemäß eingesetzten Verbindung (A) kann es sich um siliciumfreie organische Verbindungen mit vorzugsweise mindestens zwei aliphatisch ungesättigten Gruppen sowie um Organosiliciumverbindungen mit vorzugsweise mindestens zwei aliphatisch ungesättigten Gruppen handeln oder auch um deren Mischungen.

Beispiele für siliciumfreie organische Verbindungen (A) sind, 1,3,5-Trivinylcyclohexan, 2,3-Dimethyl-1,3-butadien, 7-Methyl-3-methylen-1,6-octadien, 2-Methyl-1,3-butadien, 1,5-Hexadien, 1,7-Octadien, 4,7-Methylen-4,7,8,9-tetrahydroinden, Methylcyclopentadien, 5-Vinyl-2-norbornen, Bicyclo[2.2.1]hepta-2,5-dien, 1,3-Diisoproppenylbenzol, vinylgruppenhaltiges Polybutadien, 1,4-Divinylcyclohexan, 1,3,5-Triallylbenzol, 1,3,5-Trivinylbenzol, 1,2,4-Trivinylcyclohexan, 1,3,5-Triisopropenylbenzol, 1,4-Divinylbenzol, 3-Methyl-heptadien-(1,5), 3-Phenyl-hexadien-(1,5), 3-Vinyl-hexadien-(1,5 und 4,5-Dimethyl-4,5-diethyl-octadien-(1,7), N,N'-Methylen-bis-acrylsäureamid, 1,1,1-Tris(hydroxymethyl)-propan-triacrylat, 1,1,1-Tris(hydroxymethyl)propantrimethacrylat, Tripropylenglykol-diacrylat, Diallylether, Diallylamin, Diallylcarbonat, N,N'-Diallylharnstoff, Triallylamin, Tris(2-methylallyl)amin, 2,4,6-Triallyloxy-1,3,5-triazin, Triallyl-s-triazin-2,4,6(1H,3H,5H)-trion, Diallylmalonsäureester, Polyethylenglykoldiacrylat, Polyethylenglykol Dimethacrylat, Poly(propylenglykol)methacrylat.

Vorzugsweise enthalten die erfindungsgemäßen Siliconzusammensetzungen als Bestandteil (A) mindestens eine aliphatisch ungesättigte Organosiliciumverbindung, wobei alle bisher in additionsvernetzenden Zusammensetzungen verwendeten, aliphatisch ungesättigten Organosiliciumverbindungen eingesetzt werden können, wie beispielsweise Silicon-Blockcopolymere mit Harnstoffsegmenten, Silicon-Blockcopolymere mit Amid-Segmenten und/oder Imid-Segmenten und/oder Ester-Amid-Segmenten und/oder Polystyrol-Segmenten und/oder Silarylen-Segmenten und/oder Carboran-Segmenten und Silicon-Pfropfcopolymere mit EtherGruppen.

Als Organosiliciumverbindungen (A), die SiC-gebundene Reste mit aliphatischen Kohlenstoff-Kohlenstoff-Mehrfachbindungen aufweisen, werden vorzugsweise lineare oder verzweigte Organopolysiloxane aus Einheiten der allgemeinen Formel (II)

RₐR⁴_{b}SiO_{(4-a-b)/2} (II)

eingesetzt, wobei
**R** unabhängig voneinander, gleich oder verschieden, ein von aliphatischen Kohlenstoff-Kohlenstoff-Mehrfachbindungen freien, organischen oder anorganischen Rest,
**R⁴** unabhängig voneinander, gleich oder verschieden einen einwertigen, substituierten oder nicht substituierten, SiC-gebundenen Kohlenwasserstoffrest mit mindestens einer aliphatischen Kohlenstoff-Kohlenstoff-Mehrfachbindung,
**a** 0, 1, 2 oder 3 ist, und
**b** 0, 1 oder 2 ist
bedeuten,
mit der Maßgabe, dass die Summe a + b kleiner oder gleich 3 ist und mindestens 2 Reste R⁴ je Molekül vorliegen.

Bei Rest R kann es sich um ein- oder mehrwertige Reste handeln, wobei die mehrwertigen Reste, wie beispielsweise bivalente, trivalente und tetravalente Reste, dann mehrere, wie etwa zwei, drei oder vier, Siloxy-Einheiten der Formel (II) miteinander verbinden.

Weitere Beispiele für R sind die einwertigen Reste -F, -Cl, - Br, OR⁵, -CN, -SCN, -NCO und SiC-gebundene, substituierte oder nicht substituierte Kohlenwasserstoffreste, die mit Sauerstoffatomen oder der Gruppe -C(O)- unterbrochen sein können, sowie zweiwertige, beidseitig gemäß Formel (II) Si-gebundene Reste. Falls es sich bei Rest R um SiC-gebundene, substituierte Kohlenwasserstoffreste handelt, sind bevorzugte Substituenten Halogenatome, phosphorhaltige Reste, Cyanoreste, -OR⁵, -NR⁵-, -NR⁵₂, -NR⁵-C(O)-NR⁵₂, -C(O)-NR⁵₂, -C(O)R⁵, -C(O)OR⁵, -SO₂-Ph und -C₆F₅. Dabei bedeuten R⁵ unabhängig voneinander, gleich oder verschieden ein Wasserstoffatom oder einen einwertigen Kohlenwasserstoffrest mit 1 bis 20 Kohlenstoffatomen und Ph gleich Phenylrest.

Beispiele für Reste R sind Alkylreste, wie der Methyl-, Ethyl-, n-Propyl-, iso-Propyl-, n-Butyl-, iso-Bulyl-, tert-Butyl-, n-Pentyl-, iso-Pentyl-, neo-Pentyl-, tert-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, und Octadecylreste, wie der n-Octadecylrest, Cycloalkylreste, wie Cyclopentyl-, Cyclohexyl-, Cycloheptyl-und Methylcyclohexylreste, Arylreste, wie der Phenyl-, Naphthyl-, Anthryl- und Phenanthrylrest, Alkarylreste, wie o-, m-, p-Tolylreste, Xylylreste und Ethylphenylreste, und Aralkylreste, wie der Benzylrest, der α- und der β-Phenylethylrest.

Beispiele für substituierte Reste R sind Halogenalkylreste, wie der 3,3,3-Trifluor-n-propylrest, der 2,2,2,2',2',2'-Hexafluorisopropylrest, der Heptafluorisopropylrest, Halogenarylreste, wie der o-, m- und p-Chlorphenylrest, -(CH₂)-N(R⁵)C(O)NR⁵₂, -(CH₂)ₙ-C(O)NR⁵₂, -(CH₂)ₙ-C(O)R⁵, -(CH₂)ₙ-C(O)OR⁵, - (CH₂)ₙ-C(O)NR⁵₂, -(CH₂)-C(O)-(CH₂)ₘC(O)CH₃, -(CH₂)-O-CO-R⁵, -(CH₂)-NR⁵-(CH₂)ₘ-NR⁵₂, -(CH₂)ₙ-O-(CH₂)ₘCH (OH) CH₂OH, - (CH₂)ₙ(OCH₂CH₂)ₘOR⁵, -(CH₂)ₙ-SO₂-Ph und - (CH₂)ₙ-O-C₆F₅, wobei R⁵ und Ph der oben dafür angegebene Bedeutung entspricht und n und m gleiche oder verschiedene ganze Zahlen zwischen 0 und 10 bedeuten.

Beispiele für R gleich zweiwertige, beidseitig gemäß Formel (II) Si-gebundene Reste sind solche, die sich von den voranstehend für Rest R genannten einwertigen Beispiele dadurch ableiten, dass eine zusätzliche Bindung durch Substitution eines Wasserstoffatoms erfolgt, Beispiele für derartige Reste sind -(CH₂)-, -CH(CH₃)-, -C(CH3)2-, -CH(CH₃)-CH₂-, -C₆H₄-, - CH(Ph)-CH₂-, -C(CF₃)₂-, -(CH₂)ₙ-C₆H₄-(CH₂)ₙ-, - (CH₂)ₙ-C₆H₄-C₆H₄-(CH₂)ₙ-, -(CH₂O)ₘ, (CH₂CH₂O)ₘ, -(CH₂)ₙ-Oₓ-C₅H₄-SO₂-C₆H₄-Oₓ-(CH₂)ₙ-, wobei x 0 oder 1 ist, und Ph, m und n die voranstehend genannte Bedeutung haben.

Bevorzugt handelt es sich bei Rest R um einen einwertigen, von aliphatischen Kohlenstoff-Kohlenstoff-Mehrfachbindungen freien, SiC-gebundenen, gegebenenfalls substituierten Kohlenwasserstoffrest mit 1 bis 18 Kohlenstoffatomen, besonders bevorzugt um einen einwertigen, von aliphatischen Kohlenstoff-Kohlenstoff-Mehrfachbindungen freien, SiC-gebundenen Kohlenwasserstoffrest mit 1 bis 6 Kohlenstoffatomen, insbesondere um den Methyl- oder Phenylrest.

Bei Rest R⁴ kann es sich um beliebige, einer Anlagerungsreaktion (Hydrosilylierung) mit einer SiHfunktionellen Verbindung zugängliche Gruppen handeln. Falls es sich bei Rest R⁴ um SiC-gebundene, substituierte Kohlenwasserstoffreste handelt, sind als Substituenten Halogenatome, Cyanoreste und -OR⁵ bevorzugt, wobei R⁵ die obengenannte Bedeutung hat.

Bevorzugt handelt es sich bei Rest R⁴ um Alkenyl- und Alkinylgruppen mit 2 bis 16 Kohlenstoffatomen, wie Vinyl-, Allyl-, Methallyl-, 1-Propenyl-, 5-Hexenyl-, Ethinyl-, Butadienyl-, Hexadienyl-, Cyclopentenyl-, Cyclopentadienyl-, Cyclohexenyl-, Vinylcyclohexylethyl-, Divinylcyclohexylethyl-, Norbornenyl-, Vinylphenyl- und Styrylreste, wobei Vinyl-, Allyl- und Hexenylreste besonders bevorzugt verwendet werden.

Das Molekulargewicht des Bestandteils (A) kann in weiten Grenzen variieren, etwa zwischen 10² und 10⁶ g/mol. So kann es sich bei dem Bestandteil (A) beispielsweise um ein relativ niedermolekulares alkenylfunktionelles Oligosiloxan, wie 1,2-Divinyltetramethyldisiloxan, handeln, jedoch auch um ein über kettenständige oder endständige Si-gebundene Vinylgruppen verfügendes hochpolymeres Polydimethylsiloxan, z.B. mit einem Molekulargewicht von 10⁵ g/mol (mittels NMR bestimmtes Zahlenmittel). Auch die Struktur der den Bestandteil (A) bildenden Moleküle ist nicht festgelegt; insbesondere kann die Struktur eines höhermolekularen, also oligomeren oder polymeren Siloxans linear, cyclisch, verzweigt oder auch harzartig, netzwerkartig sein. Lineare und cyclische Polysiloxane sind vorzugsweise aus Einheiten der Formel R₃SiO_{1/2}, R⁴R₂SiO_{2/2}, R⁴RSiO_{1/2} und R₂SiO_{2/2} zusammengesetzt, wobei R und R⁴ die vorstehend angegebene Bedeutung haben. Verzweigte und netzwerkartige Polysiloxane enthalten zusätzlich trifunktionelle und/oder tetrafunktionelle Einheiten, wobei solche der Formeln RSiO_{3/2}, R⁴SiO_{3/2} und SiO_{4/2} bevorzugt sind. Selbstverständlich können auch Mischungen unterschiedlicher, den Kriterien des Bestandteils (A) genügender Siloxane eingesetzt werden.

Besonders bevorzugt als Komponente (A) ist die Verwendung vinylfunktioneller, im wesentlichen linearer Polydiorganosiloxane mit einer Viskosität von 0,01 bis 500 000 Pa·s, besonders bevorzugt von 0,1 bis 100 000 Pa·s, jeweils bei 25°C.

Als Organosiliciumverbindung (B) können alle hydrogenfunktionellen Organosiliciumverbindungen eingesetzt werden, die auch bisher in additionsvernetzbaren Zusammensetzungen eingesetzt worden sind.

Als Organopolysiloxane (B), die Si-gebundene Wasserstoffatome aufweisen, werden vorzugsweise lineare, cyclische oder verzweigte Organopolysiloxane aus Einheiten der allgemeinen Formel (III)

R_{c}H_{d}SiO_{(4-c-d)/2} (III)

eingesetzt, wobei
**R** die oben angegebene Bedeutung hat,
**c** 0,1 2 oder 3 ist und
**d** 0, 1 oder 2 ist,
mit der Maßgabe, dass die Summe von c + d kleiner oder gleich 3 ist und mindestens zwei Si gebundene Wasserstoffatome je Molekül vorliegen.

Vorzugsweise enthält das erfindungsgemäß eingesetzte Organopolysiloxan (B) Si-gebundenen Wasserstoff im Bereich von 0,04 bis 1,7 Gewichtsprozent, bezogen auf das Gesamtgewicht des Organopolysiloxans (B).

Das Molekulargewicht des Bestandteils (B) kann ebenfalls in weiten Grenzen variieren, etwa zwischen 10² und 10⁶ g/mol. So kann es sich bei dem Bestandteil (B) beispielsweise um ein relativ niedermolekulares SiH-funktionelles Oligosiloxan, wie Tetramethyldisiloxan, handeln, jedoch auch um ein über kettenständig oder endständig SiH-Gruppen verfügendes hochpolymeres Polydimethylsiloxan oder ein SiH-Gruppen aufweisendes Siliconharz.

Auch die Struktur der den Bestandteil (B) bildenden Moleküle ist nicht festgelegt; insbesondere kann die Struktur eines höhermolekularen, also oligomeren oder polymeren SiH-haltigen Siloxans linear, cyclisch, verzweigt oder auch harzartig, netzwerkartig sein. Lineare und cyclische Polysiloxane (B) sind vorzugsweise aus Einheiten der Formel R₃SiO_{1/2}, HR₂SiO_{1/2}, HRSiO_{2/2} und R₂SiO_{2/2} zusammengesetzt, wobei R die vorstehend angegebene Bedeutung hat. Verzweigte und netzwerkartige Polysiloxane enthalten zusätzlich trifunktionelle und/oder tetrafunktionelle Einheiten, wobei solche der Formeln RSiO_{3/2}, HSiO_{3/2} und SiO_{4/2} bevorzugt sind, wobei R die vorstehend angegebene Bedeutung hat.

Selbstverständlich können auch Mischungen unterschiedlicher, den Kriterien des Bestandteils (B) genügender Siloxane eingesetzt werden. Insbesondere können die den Bestandteil (B) bildenden Moleküle zusätzlich zu den obligaten SiH-Gruppen ggf. zugleich auch aliphatisch ungesättigte Gruppen enthalten. Besonders bevorzugt ist die Verwendung niedermolekularer SiHfunktioneller Verbindungen wie Tetrakis(dimethylsiloxy)silan und Tetramethylcyclotetrasiloxan, sowie höhermolekularer, SiHhaltiger Siloxane, wie Poly(hydrogenmethyl)siloxan und Poly(dimethylhydrogenmethyl)siloxan mit einer Viskosität bei 25°C von 10 bis 10 000 mPa•s, oder analoge SiH-haltige Verbindungen, bei denen ein Teil der Methylgruppen durch 3,3,3-Trifluorpropyl- oder Phenylgruppen ersetzt ist.

Bestandteil (B) ist vorzugsweise in einer solchen Menge in den erfindungsgemäßen vernetzbaren Siliconzusammensetzungen enthalten, dass das Molverhältnis von SiH-Gruppen zu aliphatisch ungesättigten Gruppen aus (A) bei 0,1 bis 20, besonders bevorzugt zwischen 1,0 und 5,0, liegt. Die erfindungsgemäß eingesetzten Komponenten (A) und (B) sind handelsübliche Produkte bzw. nach in der Chemie gängigen Verfahren herstellbar.

Anstelle von Komponente (A) und (B) können die erfindungsgemäßen Siliconzusammensetzungen Organopolysiloxane (C), die gleichzeitig aliphatische Kohlenstoff-Kohlenstoff-Mehrfachbindungen und Si-gebundene Wasserstoffatome aufweisen, enthalten. Auch können die erfindungsgemäßen Siliconzusammensetzungen alle drei Komponenten (A), (B) und (C) enthalten.

Falls Siloxane (C) eingesetzt werden, handelt es sich vorzugsweise um solche aus Einheiten der allgemeinen Formeln (IV), (V) und (VI)

R_{f}SiO_{4-f/2} (IV)

R_{g}R⁴SiO_{3-g/2} (V)

RₕHSiO_{3-h/2} (VI)

wobei
**R** und **R⁴** die oben dafür angegebene Bedeutung haben
**f** 0, 1, 2 oder 3 ist,
**g** 0, 1 oder 2 ist und
**h** 0, 1 oder 2 ist,
mit der Maßgabe, dass je Molekül mindestens 2 Reste R⁴ und mindestens 2 Si-gebundene Wasserstoffatome vorliegen.

Beispiele für Organopolysiloxane (C) sind solche aus SiO_{4/2}, R₃SiO_{1/2}-, R₂R⁴SiO_{1/2}- und R₂HSiO_{1/2}- Einheiten, sogenannte MQ-Harze, wobei diese Harze zusätzlich RSiO_{3/2}- und R₂SiO-Einheiten enthalten können, sowie lineare Organopolysiloxane im wesentlichen bestehend aus R₂R⁴SiO_{1/2}-, R₂SiO- und RHSiO-Einheiten mit R und R¹¹ gleich der obengenannten Bedeutung.

Die Organopolysiloxane (C) besitzen vorzugsweise eine durchschnittliche Viskosität von 0,01 bis 500 000 Pa·s, besonders bevorzugt 0,1 bis 100 000 Pa·s jeweils bei 25°C. Organopolysiloxane (C) sind nach in der Chemie gängigen Methoden herstellbar.

Erfindungsgemäße additionsvernetzende Siliconzusammensetzungen, werden ausgewählt aus der Gruppe enthaltend
- jeweils mindestens eine Verbindung (A), (B) und (D),
- jeweils mindestens eine Verbindung (C) und (D), und
- jeweils mindestens eine Verbindung (A), (B), (C) und (D) wobei
   (A) eine organische Verbindung oder eine Siliziumorganische Verbindung, enthaltend mindestens zwei Reste mit aliphatischen Kohlenstoff-Kohlenstoff-Mehrfachbindungen,
   (B) ein Siliziumorganische Verbindung, enthaltend mindestens zwei Si-gebundenen Wasserstoffatome,
   (C) eine Siliziumorganische Verbindung, enthaltend SiC-gebundene Reste mit aliphatischen Kohlenstoff-Kohlenstoff-Mehrfachbindungen und Si- gebundene Wasserstoffatome, und
   (D) ein Platinkatalysator
   bedeuten,
wobei der Platinkatalysator (D) der folgenden Definition entspricht.

Ein weiterer Gegenstand der Erfindung ist die Komponente (D), da sie ausschlaggebend für die Eigenschaften der erfindungsgemäßen Siliconzusammensetzungen ist.

Der erfindungsgemäße Platinkatalysator (D) entspricht der allgemeinen Formel (I),

R¹₂Pt[P(OR²)₃]₂ (I)

wobei
R¹ unabhängig voneinander, gleich oder verschieden, ein
   - Halogen,
   - einfach negativer anorganischer Rest,
   - lineare oder verzweigte aliphatische Reste der allgemeinen Formeln CᵥH₂ᵥ₊₁ oder CᵥH₂ᵥ₋₁ mit v = 1 bis 18, Arylalkylreste mit 6 bis 31 Kohlenstoffatomen
   - -O-Alkyl, -O-Aryl, -O-Aryl-Alkyl,
   - -Si(Alkyl)₃, -Si(Aryl)₃, Si(Aryl-Alkyl),
R² unabhängig voneinander, gleich oder verschieden ein Arylalkyl der Formel -(C₆H₅₋p)-(CₒH₂ₒ₊₁)ₚ mit o = 1 - 31 und p = 1 - 5,
wobei mindestens ein Alkylsubstituent an der 2-Position des Phenylrings gebunden ist,
wobei die Wasserstoffatome der für R¹ und R² oben genannten Verbindungen substituiert oder nicht substituiert sind durch die Gruppen -NH₂, -COOH, -F, -Br, -C1, -Aryl oder -Alkyl
bedeuten.

(D) stellt eine speziell hergestellte Platin-Komplexverbindung dar. Das Herstellverfahren erfolgt durch die Umsetzung eines Platinsalzes wie beispielsweise K₂PtCl₄, Na₂PtCl₄, PtCl₂, PtBr₂ oder PtI₂, mit dem jeweiligen Phosphit der Formel [P(OR²)₃], wobei R² die oben genannte Bedeutung trägt, in einem für die Reaktion geeigneten Lösungsmittel bei einer Temperatur von 0 bis 110 °C dargestellt wird. Die Herstellung der eingesetzten Phosphite für diese Reaktion entspricht gängigen Verfahren aus dem Stand der Technik oder sie sind kommerziell erhältlich. Vor der Einmischung in die erfindungsgemäße Siliconzusammensetzung wird die Verbindung (D) isoliert und ihre Reinheit mit Hilfe gängiger Methoden überprüft. Das eingesetzte Phosphit der Formel [P(OR²)₃] koordiniert an das Zentralmetall, wobei R² die oben genannte Bedeutung trägt.

Anbei folgt eine beispielhafte Auflistung erfindungsgemäßer Platin-Phosphitkomplexe [D], bei denen R¹ = Cl ist, R² variiert wurde und die auf dem oben beschriebenen Weg synthetisiert wird.
PtCl₂[P(-O-2-methylphenyl)₃]₂,
PtCl₂[P(-O-2-ethylphenyl)₃]₂,
PtCl₂[P(-O-2-propylphenyl)₃]₂,
PtCl₂[P(-O-2-isopropylphenyl)₃]₂,
PtCl₂[P(-O-2-butylphenyl)₃]₂,
PtCl₂[P(-O-2-sec-butylphenyl)₃]₂,
PtCl₂[P(-O-2-tert-butylphenyl)₃]₂,
PtCl₂[P(-O-2-pentylphenyl)₃]₂,
PtCl₂{P[-O-2-(1-methylbutyl)phenyl]₃}₂,
PtCl₂[P(-O-2-hexylphenyl)₃]₂,
PtCl₂[P(-O-2-heptylphenyl)₃]₂,
PtCl₂[P(-O-2-octylphenyl)₃]₂,
PtCl₂[P(-O-2-nonylphenyl)₃]₂,
PtCl₂[P(-O-2-decylphenyl)₃]₂,
PtCl₂[P(-O-2-octadecylphenyl)₃]₂,
PtCl₂[P(-O-2-octadecenylphenyl)₃]₂,
PtCl₂{P[-O-2-(1,1-dimethylpropyl)phenyl]₃}₂,
PtCl₂{P[-O-2-(1,1-dimethylbutyl)phenyl]₃}₂,
PtCl₂{P[-O-2-(1,1-dimethylpentyl)phenyl]₃}₂,
PtCl₂{P[-O-2-(1,1-dimethylhexyl)phenyl]₃}₂,
PtCl₂{P[-O-2-(1,1-dimethylheptyl)phenyl]₃}₂,
PtCl₂{P[-O-2-(1,1,3,3-tetramethylbutyl)phenyl]₃}₂,

PtCl₂[P(-O-2,4-dimethylphenyl)₃]₂,
PtCl₂[P(-O-2,4-diethylphenyl)₃]₂,
PtCl₂[P(-O-2,4-dipropylphenyl)₃]₂,
PtCl₂[P(-O-2,4-diisopropylphenyl)₃]₂,
PtCl₂[P(-O-2,4-dibutylphenyl)₃]₂,
PtCl₂[P(-O-2,4-di-sec-butylphenyl)₃]₂,
PtCl₂[P(-O-2,4-di-tert-butylphenyl)₃]₂,
PtCl₂[P(-O-2,4-di-pentylphenyl)₃]₂,
PtCl₂{P[-O-2,4-bis(1-methylbutyl)phenyl]₃}₂,
PtCl₂[P(-O-2,4-dihexylphenyl)₃]₂,
PtCl₂[P(-O-2,4-diheptylphenyl)₃]₂,
PtCl₂[P(-O-2,4-dioctylphenyl)₃]₂,
PtCl₂[P(-O-2,4-dinonylphenyl)₃]₂,
PtCl₂[P(-O-2,4-didecylphenyl)₃]₂,
PtCl₂[P(-O-2,4-dioctadecylphenyl)₃]₂,
PtCl₂[P(-O-2,4-dioctadecenylphenyl)₃]₂,
PtCl₂{P[-O-2,4-bis(1,1-dimethylpropyl)phenyl]₃}₂,
PtCl₂{P[-O-2,4-bis(1,1-dimethylbutyl)phenyl]₃}₂,
PtCl₂{P[-O-2,4-bis(1,1-dimethylpentyl)phenyl]₃}₂,
PtCl₂{P[-O-2,4-bis(1,1-dimethylhexyl)phenyl]₃}₂,
PtCl₂{P[-O-2,4-bis(1,1-dimethylheptyl)phenyl]₃}₂,
PtCl₂{P[-O-2,4-bis(1,1,3,3-tetramethylbutyl)phenyl]₃}₂,

PtCl₂[P(-O-2,5-dimethylphenyl)₃]₂,
PtCl₂[P(-O-2,5-diethylphenyl)₃]₂,
PtCl₂[P(-O-2,5-dipropylphenyl)₃]₂,
PtCl₂[P(-O-2,5-diisopropylphenyl)₃]₂,
PtCl₂[P(-O-2,5-dibutylphenyl)₃]₂,
PtCl₂[P(-O-2,5-di-sec-butylphenyl)₃]₂,
PtCl₂[P(-O-2,5-di-tert-butylphenyl)₃]₂,
PtCl₂[P(-O-2,5-di-pentylphenyl)₃]₂,
PtCl₂{P[-O-2,5-bis(1-methylbutyl)phenyl]₃}₂,
PtCl₂[P(-O-2,5-dihexylphenyl)₃]₂,
PtCl₂[P(-O-2,5-diheptylphenyl)₃]₂,
PtCl₂[P(-O-2,5-dioctylphenyl)₃]₂,
PtCl₂[P(-O-2,5-dinonylphenyl)₃]₂,
PtCl₂[P(-O-2,5-didecylphenyl)₃]₂,
PtCl₂[P(-O-2,5-dioctadecylphenyl)₃]₂,
PtCl₂[P(-O-2,5-dioctadecenylphenyl)₃]₂,
PtCl₂{P[-O-2,5-bis(1,1-dimethylpropyl)phenyl]₃}₂,
PtCl₂{P[-O-2,5-bis(1,1-dimethylbutyl)phenyl]₃}₂,
PtCl₂{P[-O-2,5-bis(1,1-dimethylpentyl)phenyl]₃}₂,
PtCl₂{P[-O-2,5-bis(1,1-dimethylhexyl)phenyl]₃}₂,
PtCl₂{P[-O-2,5-bis(1,1-dimethylheptyl)phenyl]₃}₂,
PtCl₂{P[-O-2,5-bis(1,1,3,3-tetramethylbutyl)phenyl]₃}₂,

PtCl₂[P(-O-2,5-dimethyl-4-methoxy-phenyl)₃]₂,
PtCl₂[P(-O-2,5-diethyl-4-methoxy-phenyl)₃]₂,
PtCl₂[P(-O-2,5-dipropyl-4-methoxy-phenyl)₃]₂,
PtCl₂[P(-O-2,5-diisopropyl-4-methoxy-phenyl)₃]₂,
PtCl₂[P(-O-2,5-dibutyl-4-methoxy-phenyl)₃]₂,
PtCl₂[P(-O-2,5-di-sec-butyl-4-methoxy-phenyl)₃]₂,
PtCl₂[P(-O-2,5-di-tert-butyl-4-methoxy-phenyl)₃]₂,
PtCl₂[P(-O-2,5-di-pentyl-4-methoxy-phenyl)₃]₂,
PtCl₂{P[-O-2,5-bis(1-methylbutyl)-4-methoxy-phenyl]₃}₂,
PtCl₂[P(-O-2,5-dihexyl-4-methoxy-phenyl)₃]₂,
PtCl₂[P(-O-2,5-diheptyl-4-methoxy-phenyl)₃]₂,
PtCl₂[P(-O-2,5-dioctyl-4-methoxy-phenyl)₃]₂,
PtCl₂[P(-O-2,5-dinonyl-4-methoxy-phenyl)₃]₂,
PtCl₂[P(-O-2,5-didecyl-4-methoxy-phenyl)₃]₂,
PtCl₂[P(-O-2,5-dioctadecyl-4-methoxy-phenyl)₃]₂,
PtCl₂[P(-O-2,5-dioctadecenyl-4-methoxy-phenyl)₃]₂,
PtCl₂{P[-O-2,5-bis(1,1-dimethylpropyl)-4-methoxy-phenyl]₃}₂,
PtCl₂{P[-O-2,5-bis(1,1-dimethylbutyl)-4-methoxy-phenyl]₃}₂,
PtCl₂{P[-O-2,5-bis(1,1-dimethylpentyl)-4-methoxy-phenyl]₃}₂,
PtCl₂{P[-O-2,5-bis(1,1-dimethylhexyl)-4-methoxy-phenyl]₃}₂,
PtCl₂{P[-O-2,5-bis(1,1-dimethylheptyl)-4-methoxy-phenyl]₃}₂,
PtCl₂{P[-O-2,5-bis(1,1,3,3-tetramethylbutyl)-4-methoxyphenyl]₃}₂,

PtCl₂[P(-O-2,5-dimethyl-4-ethoxy-phenyl)₃]₂,
PtCl₂[P(-O-2,5-diethyl-4-ethoxy-phenyl)₃]₂,
PtCl₂[P(-O-2,5-dipropyl-4-ethoxy-phenyl)₃]₂,
PtCl₂[P(-O-2,5-diisopropyl-4-ethoxy-phenyl)₃]₂,
PtCl₂[P(-O-2,5-dibutyl-4-ethoxy-phenyl)₃]₂,
PtCl₂[P(-O-2,5-di-sec-butyl-4-ethoxy-phenyl)₃]₂,
PtCl₂[P(-O-2,5-di-tert-butyl-4-ethoxy-phenyl)₃]₂,
PtCl₂[P(-O-2,5-di-pentyl-4-ethoxy-phenyl)₃]₂,
PtCl₂{P[-O-2,5-bis(1-methylbutyl)-4-ethoxy-phenyl]₃}₂,
PtCl₂[P(-O-2,5-dihexyl-4-ethoxy-phenyl)₃]₂,
PtCl₂[P(-O-2,5-diheptyl-4-ethoxy-phenyl)₃]₂,
PtCl₂[P(-O-2,5-dioctyl-4-ethoxy-phenyl)₃]₂,
PtCl₂[P(-O-2,5-dinonyl-4-ethoxy-phenyl)₃]₂,
PtCl₂[P(-O-2,5-didecyl-4-ethoxy-phenyl)₃]₂,
PtCl₂[P(-O-2,5-dioctadecyl-4-ethoxy-phenyl)₃]₂,
PtCl₂[P(-O-2,5-dioctadecenyl-4-ethoxy-phenyl)₃]₂,
PtCl₂{P[-O-2,5-bis(1,1-dimethylpropyl)-4-ethoxy-phenyl]₃}₂,
PtCl₂{P[-O-2,5-bis(1,1-dimethylbutyl)-4-ethoxy-phenyl]₃}₂,
PtCl₂{P[-O-2,5-bis(1,1-dimethylpentyl)-4-ethoxy-phenyl]₃}₂,
PtCl₂{P[-O-2,5-bis(1,1-dimethylhexyl)-4-ethoxy-phenyl]₃}₂,
PtCl₂{P[-O-2,5-bis(1,1-dimethylheptyl)-4-ethoxy-phenyl]₃}₂,
PtCl₂{P[-O-2,5-bis(1,1,3,3-tetramethylbutyl)-4-ethoxyphenyl]₃}₂,

PtCl₂{P[(-O-2-tert-butyl-5-methylphenyl)(-O-2,4-di-tert-butylphenyl)₂] }₂,
PtCl₂{P[(-O-2,4-di-tert-pentylphenyl)(-O-2,4-di-tert-butylphenyl)₂] }₂,
PtCl₂{P[(-O-2-tert-butylphenyl)(-O-2,4-di-tert-butylphenyl)₂]}₂,

Die erfindungsgemäßen Platinkatalysatoren (D) sind nicht auf die voran genannten Beispiele beschränkt sind, da für R¹ eine Vielzahl von Substituent einsetzbar ist. Die Reste R¹ können unabhängig voneinander einwertige Reste sein, die in der Lage sind, aus dem Zentralmetall Platin in der Oxidationsstufe + II, das zwei Phosphitliganden trägt, in Summe einen ungeladenen Komplex zu formen.

Beispiele für R¹ als einfach negativer anorganischer Reste, sind Pseudohalogenide ausgewählt aus der Gruppe enthaltend N₃⁻, CN⁻, OCN⁻, CNO⁻, SCN⁻, NCS⁻, SeCN⁻.

Bevorzugt sind als Reste R¹ Halogene, Pseudohalogene und Alkylresten.

Beispiele für R¹ sind neben dem -Cl, auch -F, -Br, -I,-CN, -N₃, -OCN, -NCO, -CNO, -SCN, -NCS, -SeCN, -CH₃, -CH₂CH₃, -CH(CH₃)₂, - C(CH₃)₃, -C₆H₅, -CH₂(Ph)-CH₃, CᵥH₂ᵥ₊₁, CᵥH₂ᵥ₋₁, -(C₆H_{5-w})-(CᵥH₂ᵥ₊₁)_{w} mit v = 1 - 18 und w = 1 - 5, -0-Alkyl, -0-Aryl, -0-Aryl-Alkyl, - Si(Alkyl)₃, -Si(Aryl)_{3,} -Si(Aryl-Alkyl)₃. Besonders bevorzugt für R¹ sind Halogene und lineare oder verzweigte aliphatische Reste mit 1 bis 18 Kohlenstoffatomen, gegebenenfalls können die darin enthaltenen H-Atome durch Gruppen wie -NH₂, -COOH, F, Br, Cl, -Alkyl, -Aryl oder -Arylalkyl ersetzt sein.

Weiterhin bevorzugt sind für R² Arylalkylreste -(C₆H₅₋ₚ)-(CₒH₂ₒ₊₁)p wobei o zwischen 1 - 18 und p zwischen 1 - 5 liegt, wobei mindestens ein Alkylsubstituent an der 2-Position des Phenylrings im Arylalkylrest gebunden. Gegenüber unsubstituierten Arylalkylphosphiten besitzen Platin-Komplexverbindungen mit substituierten Phosphiten den Vorteil einer wesentlich niedrigeren Anspringtemperatur.

Die erfindungsgemäßen Platinkatalysatoren (D) sind nützlich als Katalysatoren für die wohlbekannte Hydrosilylierungsreaktion in der Organosiliciumchemie, als Katalysator für die Hydrierung ungesättigter organischer Verbindungen oder Polymere und zur Oligomerisierung von Acetylen und anderen Alkinen.

Die erfindungsgemäßen Platinkatalysatoren (D) haben des Weiteren den Vorteil, dass endständige Doppelbindungen bei der Hydrosilylierung nicht nach innen umlagern, wodurch schwachreaktives isomerisiertes Ausgangsprodukt verblieben.

Die erfindungsgemäßen Platin-Katalysatoren haben ferner den Vorteil, dass keine Platin-Kolloide gebildet werden und durch ihren Einsatz keine Verfärbungen resultieren.

Neben den oben genannten Komponenten (A), (B), (C) und (D) können noch weitere Komponenten (E), (F) oder (G) in den erfindungsgemäßen Siliconzusammensetzungen enthalten sein.

Komponenten (E) wie beispielsweise Inhibitoren und Stabilisatoren dienen der gezielten Einstellung der Verarbeitungszeit, Anspringtemperatur und Vernetzungsgeschwindigkeit der erfindungsgemäßen Siliconzusammensetzungen. Diese Inhibitoren und Stabilisatoren sind auf dem Gebiet der additionsvernetzenden Zusammensetzungen sehr gut bekannt. Beispiele gebräuchlicher Inhibitoren sind acetylenische Alkohole, wie 1-Ethinyl-1-cyclohexanol, 2-Methyl-3-butin-2-ol und 3.5-Dimethyl-1-hexin-3-ol, 3-Methyl-1-dodecin-3-ol, Polymethylvinylcyclosiloxane wie 1,3,5,7-Tetravinyltetramethyltetracyclosiloxan niedermolekulare Siliconöle mit Methylvinyl-SiO_{1/2}-Gruppen und/oder R₂vinylSiO_{1/2}-Endgruppen. wie Divinyltetramethydisiloxan Tetravinyldimethyldisiloxan, Trialkylcyanurate, Alkylmaleate, wie Diallylmaleate, Dimethylmaleat und Diethylmaleat, Alkylfumarate, wie Diallylfumarat und Diethylfurnarat, organische Hydroperoxide wie Cumolhydroperoxid, tert-Butylhydroperoxid und Pinanhydroperoxid, organische Peroxide, organische Sulfoxide organische Amine. Diamine und Amide, Phosphane und Phosphite, Nitrile, Triazole, Diaziridine und Oxime. Die Wirkung dieser Inhibitorzusätze (E) hängt von ihrer chemischen Struktur ab, so dass die Konzentration individuell bestimmt werden muss. Inhibitoren und Inhibitormischungen werden vorzugsweise in einem Mengenanteil von 0,00001 % bis 5 % bezogen auf das Gesamtgewicht der Mischung zugesetzt, bevorzugt 0,00005 bis 2 % und besonders bevorzugt 0,0001 bis 1 %.

Komponenten (F) sind alle weiteren Zusatzstoffe Stoffe, die auch bisher zur Herstellung von additionsvernetzbaren Zusammensetzungen eingesetzt wurden. Beispiele für verstärkende Füllstoffe, die als Komponente (F) in den erfindungsgemäßen Siliconzusammensetzungen eingesetzt werden können, sind pyrogene oder gefällte Kieselsäuren mit BET-Oberflächen von mindestens 50 m²/g sowie Ruße und Aktivkohlen wie Furnace-Ruß und Acetylen-Ruß, wobei pyrogene und gefällte Kieselsäuren mit BET-Oberflächen von mindestens 50 m²/g bevorzugt sind. Die genannten Kieselsäurefüllstoffe können hydrophilen Charakter haben oder nach bekannten Verfahren hydrophobiert sein. Beim Einmischen hydrophiler Füllstoffe ist die Zugabe eines Hydrophobierungsmittels erforderlich. Der Gehalt der erfindungsgemäßen vernetzbaren Zusammensetzung an aktiv verstärkendem Füllstoff (F) liegt im Bereich von 0 bis 70 Gew.-%, vorzugsweise bei 0 bis 50 Gew.-%.

Die erfindungsgemäße Siliconzusammensetzung kann wahlweise die Komponente (F) als weitere Zusätze zu einem Anteil von bis zu 70 Gew.-%, vorzugsweise 0,0001 bis 40 Gew.-%, enthalten. Diese Zusätze können z.B. inaktive Füllstoffe, harzartige Polyorganosiloxane, die von den Siloxanen (A), (B) und (C) verschieden sind, verstärkende und nicht verstärkende Füllstoffe, Fungizide, Duftstoffe, rheologische Additive, Korrosionsinhibitoren, Oxidationsinhibitoren, Lichtschutzmittel, flammabweisend machende Mittel und Mittel zur Beeinflussung der elektrischen Eigenschaften Dispergierhilfsmittel, Lösungsmittel, Haftvermittler, Pigmente, Farbstoffe, Weichmacher, organische Polymere, Hitzestabilisatoren usw. sein. Hierzu zählen Zusätze, wie Quarzmehl, Diatomeenerde, Tone, Kreide, Lithopone, Ruße, Graphit, Metalloxide, Metallcarbonate, -sulfate, Metallsalze von Carbonsäuren, Metallstäube, Fasern, wie Glasfasern, Kunststoffasern, Kunststoffpulver, Metallstäube, Farbstoffe, Pigmente usw.

Die erfindungsgemäße Siliconzusammensetzung kann wahlweise als weitere Komponente (G) mindestens einen weiteren additionsvernetzenden Katalysatore enthalten, der dem Stand der Technik entspricht, beispielsweise Hydrosilylierungskatalysatoren oder Peroxide.

Beispiele für solche Katalysatoren (G) sind metallisches und feinverteiltes Platin, das sich auf Trägern, wie Siliciumdioxid, Aluminiumoxyd oder Aktivkohle befinden kann, Verbindungen oder Komplexe von Platin, wie Platinhalogenide, z.B. PtCl4, H2PtCl6*6H2O, Na2PtCl4*4H2O, Platin-Olefin-Komplexe, Platin-Alkohol-Komplexe, Platin-Alkoholat-Komplexe, Platin-Ether-Komplexe, Platin-Aldehyd-Komplexe, Platin-Keton-Komplexe, einschließlich Umsetzungsprodukten aus H2PtCl6*6H2O und Cyclohexanon, Platin-Vinylsiloxankomplexe, wie Platin-1,3-Divinyl-1,1,3,3-tetra-methyl-disiloxankomplexe mit oder ohne Gehalt an nachweisbarem anorganisch gebundenem Halogen, Bis-(gamma-picolin)-platindichlorid, Trimethylendipyridinplatindichlorid, Dicyclopentadienplatindichlorid, Dimethylsulfoxydethylenplatin-(II)-di-chlorid, Cyclooctadien-Platindichlorid, Norbornadien-Platindichlorid, Gamma-picolin-Platindichlorid, Cyclopentadien-Platindichlorid, sowie Umsetzungsprodukte von Platintetrachlorid mit Olefin und primärem Amin oder sekundärem Amin oder primärem und sekundärem Amin, wie das Umsetzungsprodukt aus in 1-Octen gelöstem Platintetrachlorid mit sec.-Butylamin oder Ammonium-Platinkomplexe.

Weiter Beispiele für solche einen Katalysator (G) sind organische Peroxide wie Acylperoxide, wie Dibenzoylperoxid, Bis-(4-chlorbenzoyl)-peroxid, Bis-(2,4-dichlorbenzoyl)-peroxid und Bis-(4-methylbenzoyl)-peroxid; Alkylperoxide und Arylperoxide, wie Di-tert.-butylperoxid, 2,5-Bis-(tert.-butylperoxy)-2,5-dimethylhexan, Dicumylperoxid und 1,3-Bis-(tert.-butylperoxy-isopropyl)-benzol; Perketale, wie 1,1-Bis-(tert.-butylperoxy)-3,3,5-trimethylcyclohexan; Perester, wie Diacetylperoxydicarbonat, tert.-Butylperbenzoat, Tert.-butylperoxy-isopropylcarbonat, Tert.-butylperoxy-isononanoat, Dicyclohexylperoxydicarbonat und 2,5-Dimethylhexan-2,5-diperbenzoat.

Die erfindungsgemäßen Siliconzusammensetzungen können, falls erforderlich, in Flüssigkeiten gelöst, dispergiert suspendiert oder emulgiert werden Die erfindungsgemäßen Zusammensetzungen können - insbesondere je nach Viskosität der Bestandteile sowie Füllstoffgehalt - niedrigviskos und gießbar sein, eine pastöse Konsistenz aufweisen pulverförmig sein oder auch geschmeidige, hochviskose Massen darstellen, wie dies bekanntermaßen bei den in Fachkreisen häufig als RTV-1, RTV-2, LSR und HTV bezeichneten Zusammensetzungen der Fall sein kann. Insbesondere können die erfindungsgemäßen Zusammensetzungen, falls sie hochviskos sind, in Form eines Granulates zubereitet werden. Hierbei kann das einzelne Granulatteilchen alle Komponenten enthalten, oder die erfindungsgemäß eingesetzten Komponenten sind getrennt in verschiedenen Granulatteilchen eingearbeitet. Hinsichtlich der elastomeren Eigenschaften der vernetzten erfindungsgemäßen Siliconzusammensetzungen wird gleichfalls das gesamte Spektrum umfasst, beginnend bei extrem weichen Silicongelen, über gummiartige Materialien bis hin zu hochvernetzten Siliconen mit glasartigem Verhalten.

Die Herstellung der erfindungsgemäßen Siliconzusammensetzungen kann nach bekannten Verfahren erfolgen, wie beispielsweise durch gleichmäßige Vermischung der einzelnen Komponenten. Die Reihenfolge dabei ist beliebig, bevorzugt ist jedoch die gleichmäßige Vermischung des Platinkatalysators (D) und gegebenenfalls (G) mit einer Mischung aus (A), (B) gegebenenfalls (E) und (F). Der erfindungsgemäß eingesetzte Platinkatalysator (D) und gegebenenfalls (G) kann dabei als Festsubstanz oder als Lösung - in einem geeigneten Lösungsmittel gelöst - oder als sogenannter Batch - gleichmäßig mit einer geringen Menge (A) oder (A) mit (E) vermischt - eingearbeitet werden.

Bei den erfindungsgemäß eingesetzten Komponenten (A) bis (G) kann es sich jeweils um eine einzelne Art einer solchen Komponente, wie auch um ein Gemisch aus mindestens zwei verschiedenen Arten einer solchen Komponente handeln. Die erfindungsgemäßen durch Anlagern von Si-gebundenem Wasserstoff an aliphatische Mehrfachbindung vernetzbaren Siliconzusammensetzungen können unter den gleichen Bedingungen vernetzt werden, wie die bisher bekannten durch Hydrosilylierungsreaktion vernetzbaren Zusammensetzungen.

Vorzugsweise handelt es sich dabei um Temperaturen von 100 bis 220°C, besonders bevorzugt von 130 bis 190°C, und einem Druck von 900 bis 1100 hPa. Es können aber auch höhere oder niedrigere Temperaturen und Drücke angewendet werden.

Ein weiterer Gegenstand der vorliegenden Erfindung sind Formkörper hergestellt durch Vernetzung der erfindungsgemäßen Zusammensetzungen.

Die erfindungsgemäßen Siliconzusammensetzungen sowie die erfindungsgemäß daraus hergestellten Vernetzungsprodukte können für alle Zwecke eingesetzt werden, für die auch bisher zu Elastomeren vernetzbare Organopolysiloxanzusammensetzungen bzw. Elastomere verwendet wurden. Dies umfasst beispielsweise die Siliconbeschichtung bzw. Imprägnierung beliebiger Substrate, die Herstellung von Formteilen, beispielsweise im Spritzgussverfahren, Vakuumextrusionsverfahren, Extrusionsverfahren Formgießen und Formpressen, und Abformungen, die Verwendung als Dicht- Einbett- und Vergussmassen usw.

Die erfindungsgemäßen vernetzbaren Siliconzusammensetzungen haben den Vorteil dass sie in einem einfachen Verfahren unter Verwendung leicht zugänglicher Ausgangsstoffe und damit wirtschaftlich hergestellt werden können. Die erfindungsgemäßen vernetzbaren Zusammensetzungen haben den weiteren Vorteil, dass sie als einkomponentige Formulierung bei 25°C und Umgebungsdruck eine gute Lagerstabilität aufweisen und erst bei erhöhter Temperatur rasch vernetzen. Die erfindungsgemäßen Siliconzusammensetzungen haben den Vorteil, dass diese bei zweikomponentiger Formulierung nach Vermischen der beiden Komponenten eine vernetzungsfähige Siliconmasse ergeben, deren Verarbeitbarkeit über einen langen Zeitraum hinweg bei 25°C und Umgebungsdruck bestehen bleibt, also extrem lange Topfzeit zeigen, und erst bei erhöhter Temperatur rasch vernetzt.

Bei der Herstellung der erfindungsgemäßen vernetzbaren Zusammensetzungen ist es von großem Vorteil dass sich der Platinkatalysator (D) gut dosieren und leicht einarbeiten lässt.

Die erfindungsgemäßen Zusammensetzungen haben des Weiteren den Vorteil, dass die daraus erhaltenen vernetzten Siliconkautschuke eine ausgezeichnete Durchsichtigkeit aufweisen.

Die erfindungsgemäßen Zusammensetzungen haben ferner den Vorteil, dass die Hydrosilylierungsreaktion sich nicht mit der Reaktionsdauer verlangsamt.

### Beispiele

In den nachstehend beschriebenen Beispielen beziehen sich alle Angaben von Teilen und Prozentsätzen, falls nicht anders angegeben, auf das Gewicht. Sofern nicht anders angegeben, werden die nachstehenden Beispiele bei einem Druck der umgebenden Atmosphäre, also etwa bei 1000 hPa, und bei Raumtemperatur, also bei etwa 20°C, bzw. bei einer Temperatur, die sich beim Zusammengeben der Reaktanden bei Raumtemperatur ohne zusätzliche Heizung oder Kühlung einstellt, durchgeführt. Im Folgenden beziehen sich alle Viskositätsangaben auf eine Temperatur von 25°C.

### Herstellung des Katalysators 1

Eine Suspension von 2,08 g K₂PtCl₄ in 40 ml abs. Ethanol und eine Lösung von 4,79 g Tris-(2-tert-butylphenyl)phosphit in 20 ml abs. Ethanol wurden unter Stickstoff vereinigt und eine Stunde zum Sieden erhitzt. Das Lösungsmittel wurde abgezogen und der Rückstand in 40 ml Diethylether aufgenommen. Es wurde dreimal mit je 20 ml Wasser ausgeschüttelt. Die organische Phase wird über Magnesiumsulfat getrocknet, über einen Faltenfilter filtriert und abgezogen. Es wurden 5,50 g eines Platinkomplexes der nachstehenden Formel isoliert:
PtCl₂[P(-O-2-tert-butylphenyl)₃]₂,

### Herstellung des Katalysators 2

Eine Suspension von 2,08 g K₂PtCl₄ in 40 ml Wasser 6,89 g Tris-(4-nonylphenyl)phosphit wurden unter Stickstoff vereinigt und eine Stunde zum Sieden erhitzt. Dabei fällt das Produkt als weißer Feststoff an. Nach der Filtration wird mit EtOH gewaschen. Es wurden 6,95 g eines Platinkomplexes der nachstehenden Formel isoliert:
PtCl₂[P(-O-4-nonylphenyl)₃]₂

### Herstellung des Katalysators 3

Eine Suspension von 1,33 g PtCl₂ in 40 ml Dichlormethan und eine Lösung von 9,05 g Tris-(1,1-dimethylbutyl-4-methoxyphenyl)phosphit in 20 ml Dichlormethan wurden unter Stickstoff vereinigt und eine Stunde zum Sieden erhitzt. Nach Abziehen des Lösungsmittels wird der Rückstand in Diethylether aufgenommen und mit Magnesiumsulfat getrocknet. Nach der Filtration über eine Glasfritte wird das Filtrat eingeengt. Es wurden 7,54 g eines Platinkomplexes der nachstehenden Formel isoliert:
PtCl₂{P[-O-2,5-bis(1,1-dimethylbutyl-4-methoxy-phenyl)]₃}₂

### Herstellung des Katalysators 4

Eine Suspension von 1,33 g PtCl₂ in 40 ml Acetonitril und eine Lösung von 6,05 g Bis(2,4-di-tert-butyl-phenyl)-(2-tert-butyl-5-methylphenyl)phosphit in 20 ml Acetonitril wurden unter Stickstoff vereinigt und eine Stunde zum Sieden erhitzt. Dabei ölt das Produkt aus. Das Lösungsmittel wird abgezogen, der ölige Rückstand in 30 ml Diethylether aufgenommen, über Natriumsulfat getrocknet. Nach der Filtration wird das Lösungsmittel abgezogen. Es wurden 5,95 g eines Platinkomplexes der nachstehenden Formel isoliert:
PtCl₂[P(-O-2,4-di-tert-butylphenyl)₂)-(-O-2-tert-butyl-5-methylphenyl)]₂

### Herstellung des Katalysators 5

Eine Suspension von 1,33 g PtCl₂ in 40 ml Acetonitril und eine Lösung von 6,47 g Tris-(2,4-di-tert-butyl-phenyl)phosphit in 20 ml Acetonitril wurden unter Stickstoff vereinigt und eine Stunde zum Sieden erhitzt. Dabei fällt das Produkt aus. Die Suspension wird auf - 20 °C abgekühlt. Nach der Filtration über einen Glasfilter wird das Produkt getrocknet. Es wurden 5,83 g eines Platinkomplexes der nachstehenden Formel isoliert:
PtCl₂[P(-O-2,4-di-tert-butylphenyl)₃]₂

### Herstellung des Katalysators 6

Eine Suspension von 2,08 g K₂PtCl₄ in 40 ml abs. Ethanol und eine Lösung von 7,31 g Tris-(isodecyl)phosphit in 20 ml abs. Ethanol wurden unter Stickstoff vereinigt und eine Stunde zum Sieden erhitzt. Das Lösungsmittel wurde abgezogen und der Rückstand in 40 ml Diethylether aufgenommen. Es wurde dreimal mit je 20 ml Wasser ausgeschüttelt. Die organische Phase wird über Magnesiumsulfat getrocknet, über einen Faltenfilter filtriert und abgezogen. Es wurden 7,15 g eines Platinkomplexes der nachstehenden Formel isoliert:
PtCl₂[P(-O-isodecyl)₃]₂

### Herstellung des Katalysators 7

Eine Suspension von 1,33 g PtCl₂ in 40 ml Acetonitril und eine Lösung von 5,63 g Tris-(2-tert-butyl-4-ethyl)phosphit in 20 ml Acetonitril wurden unter Stickstoff vereinigt und eine Stunde zum Sieden erhitzt. Dabei fällt das Produkt aus. Die Suspension wird auf - 20 °C abgekühlt. Nach der Filtration über einen Glasfilter wird das Produkt getrocknet. Es wurden 5,83 g eines Platinkomplexes der nachstehenden Formel isoliert:
PtCl₂[P(-O-2-tert-butyl-4-ethyl-phenyl)₃]₂

### Herstellung des Katalysators 8

Zu 2,0 g einer Suspension von Katalysator 2 in 30 ml abs. Diethylether werden bei -20 °C 5 ml einer 1,0 molaren Lösung von Trimethylaluminium in Diethylether gegeben. Die Reaktionsmischung wird nach Erwärmen bei Raumtemperatur eine Stunde gerührt. Nach vorsichtiger Zugabe von 20 ml Wasser wird die Etherphase abdekantiert. Die wässrige Phase wird dreimal mit jeweils 20 ml Diethylether ausgeschüttelt. Die vereinigten organischen Extrakte werden über wasserfreiem Natriumsulfat getrocknet. Nach Abziehen des Lösungsmittels erhält man 1,5 g einer Verbindung der Formel:
Me₂PtPt[P(-O-4-nonylphenyl)₃]₂

### Herstellung des Katalysators 9

Zu 2,0 g einer Suspension von Katalysator 5 in 30 ml abs. Diethylether werden bei -20 °C 7,2 ml einer 2,0 molaren Lösung von Methyllithium Diethylether gegeben. Die Reaktionsmischung wird nach Erwärmen eine Stunde bei Raumtemperatur gerührt. Nach vorsichtiger Zugabe von 20 ml Wasser wird die Etherphase abdekantiert. Die wässrige Phase wird dreimal mit jeweils 20 ml Diethylether ausgeschüttelt. Die vereinigten organischen Extrakte werden über wasserfreiem Natriumsulfat getrocknet. Nach Abziehen des Lösungsmittels erhält man 1,7 g einer Verbindung der Formel:
Me₂PtP(-O-2,4-di-tert-butylphenyl)₃]₂

### Herstellung des Katalysators 10

Zu 2,0 g einer Suspension von Katalysator 5 in 30 ml abs. Diethylether werden bei -20 °C 7,2 ml einer 2,0 molaren Lösung von Butyllithium Diethylether gegeben. Die Reaktionsmischung nach Erwärmen eine Stunde bei Raumtemperatur gerührt. Nach vorsichtiger Zugabe von 20 ml Wasser wird die Etherphase abdekantiert. Die wässrige Phase wird dreimal mit jeweils 20 ml Diethylether ausgeschüttelt. Die vereinigten organischen Extrakte werden über wasserfreiem Natriumsulfat getrocknet. Nach Abziehen des Lösungsmittels erhält man 2,1 g einer Verbindung der Formel:
Butyl₂PtP(-O-2,4-di-tert-butylphenyl)₃]₂

### Beispiel 1

Allgemeine Verfahrensweise: 50,0 g eines Vinyldimethylsiloxyterminierten Polydimethylsiloxans mit einer Viskosität von 20 Pa•s, Inhibitor und 1,0 g SiH-Vernetzer wurden mit Hilfe eines Rührers der Firma Janke & Kunkel IKA-Labortechnik, Typ RE 162 homogen vermischt, wobei der SiH-Vernetzer ein Mischpolymerisat aus Dimethylsiloxy- und Methylhydrogensiloxy- und Trimethylsiloxy-Einheiten mit einer Viskosität von 330 mPa•s und einem Gehalt an Si-gebundenem Wasserstoff von 0,46 Gew.% war. Anschließend wurden 10 ppm Platinkomplex (bezogen auf Pt) in 0,5 ml Dichlormethan gelöst, zugegeben und bei Raumtemperatur eingerührt.

In Beispiel 1 wurden 3 mg 1-Ethinyl-1-cyclohexanol (ECH) als Inhibitorkomponente und 3,2 mg Katalysator 1 (entspricht 10 ppm Pt) verwendet.

### Beispiel 2

Die in Beispiel 1 beschriebene Verfahrensweise wird wiederholt mit dem Unterschied, dass als Inhibitorkomponente 20,0 mg 2-Phenyl-3-butin-2-ol eingesetzt werden. Dazu werden 3,2 mg Katalysator 1 eingerührt.

### Beispiel 3

Die in Beispiel 1 beschriebene Verfahrensweise wird wiederholt mit dem Unterschied, dass als Inhibitorkomponente 3 mg Diethylmaleat eingesetzt werden. Dazu werden 3,2 mg Katalysator 1 eingerührt.

### Beispiel 4

Die in Beispiel 1 beschriebene Verfahrensweise wird wiederholt mit dem Unterschied, dass als Inhibitorkomponente eine Kombination aus 2,0 mg Tris(2,4-di-tert-butylphenyl) phosphit und 2,0 mg Diethylmaleat eingesetzt wird. Dazu werden 3,2 mg Katalysator 1 eingerührt.

### Referenz Beispiel 5

Die in Beispiel 1 beschriebene Verfahrensweise wird wiederholt mit dem Unterschied, dass als Inhibitorkomponente eine Kombination aus 1,0 mg Tris(2,4-di-tert-butylphenyl) phosphit und 1,0 mg Diethylmaleat eingesetzt wird. Dazu werden 4,3 mg Katalysator 2 eingerührt.

### Beispiel 6

Die in Beispiel 1 beschriebene Verfahrensweise wird wiederholt mit dem Unterschied, dass als Inhibitorkomponente 3 mg ECH eingesetzt werden. Dazu werden 5,0 mg Katalysator 3 eingerührt.

### Beispiel 7

Die in Beispiel 1 beschriebene Verfahrensweise wird wiederholt mit dem Unterschied, dass als Inhibitorkomponente eine Kombination aus 1,0 mg Tris(2,4-di-tert-butylphenyl) phosphit und 1,0 mg Diethylmaleat eingesetzt wird. Dazu werden 3,9 mg Katalysator 4 eingerührt.

### Beispiel 8

Die in Beispiel 1 beschriebene Verfahrensweise wird wiederholt mit dem Unterschied, dass als Inhibitorkomponente eine Kombination aus 1,0 mg Tris(2,4-di-tert-butylphenyl) phosphit und 1,0 mg Diethylmaleat eingesetzt wird. Dazu werden 4,1 mg Katalysator 5 eingerührt.

### Referenz Beispiel 9

Die in Beispiel 1 beschriebene Verfahrensweise wird wiederholt mit dem Unterschied, dass als Inhibitorkomponente 3 mg ECH eingesetzt werden. Dazu werden 3,3 mg Katalysator 6 eingerührt.

### Beispiel 10

Die in Beispiel 1 beschriebene Verfahrensweise wird wiederholt mit dem Unterschied, dass als Inhibitorkomponente 3 mg ECH eingesetzt werden. Dazu werden 3,6 mg Katalysator 7 eingerührt.

### Referenz Beispiel 11

Die in Beispiel 1 beschriebene Verfahrensweise wird wiederholt mit dem Unterschied, dass als Inhibitorkomponente 3 mg ECH eingesetzt werden. Dazu werden 3,8 mg Katalysator 8 eingerührt.

### Beispiel 12

Die in Beispiel 1 beschriebene Verfahrensweise wird wiederholt mit dem Unterschied, dass als Inhibitorkomponente eine Kombination aus 1,0 mg Tris(2,4-di-tert-butylphenyl) phosphit und 1,0 mg Diethylmaleat eingesetzt wird. Dazu werden 3,8 mg Katalysator 9 eingerührt.

### Vergleichsbeispiel 1:

Als Vergleichsbeispiel ist die Vernetzung mit einem Platindivinyltetramethylsiloxan-Komplex (1) angeführt, das trotz Zugabe inhibierender Substanzen wie ECH bei 50 °C eine kurze Topfzeit von unter einem Tag aufweist.

Die Topfzeiten wurden durch visuelle Beurteilung einer niedrigviskosen Modellrezeptur ermittelt, die Anspringtemperaturen sind von den gewählten Methodenparametern abhängig und wurden mit Hilfe einer an DIN53529T3 angelehnten Methode bestimmt.

Es werden folgende Abkürzungen verwendet:
- Bsp: Beispiel
- Kat: Katalysator
- Inh: Inhibitor
- ECH: 1-Ethinyl-1-cyclohexanol
- Temp: Anspringtemperatur
- Δt: Topfzeit bei 50 °C
- Kat 0: Platindivinyltetramethylsiloxan-Komplex, "Karstedt- Kat"
- Vgl 1: Vergleichsbeispiel 1

In Tabelle 1 sind die Anspringtemperaturen und Topfzeiten der Beispiele 1 - 10 sowie des Vergleichsbeispiels wiedergegeben. Es zeigte sich, dass eine Topfzeit von mindestens 2 Tagen erreicht werden konnte.

**Tabelle 1**

| **Bsp** | **Kat** | **Inh** | **Temp** | **Δt** |
|---|---|---|---|---|
| Vgl 1 | 0 | 1-Ethinyl-1-cyclohexanol | 103 | < 1 Tag |
| 2 | 1 | 1-Ethinyl-1-cyclohexanol | 119 | 4 Tage |
| 3 | 1 | 2-Phenyl-3-butin-2-ol | 125 | 3 Tage |
| 4 | 1 | Diethylmaleat | 120 | 2 Tage |
| 5 | 1 | Tris(2,4-di-tert-butylphenyl)phosphit, Diethylmaleat | 119 | > 6 Tage |
| 6 | 2 | Tris(2,4-di-tert-butylphenyl)phosphit, Diethylmaleat | 148 | > 6 Tage |
| 7 | 3 | 1-Ethinyl-1-cyclohexanol | 144 | 4 Tage |
| 8 | 4 | Tris(2,4-di-tert-butylphenyl)phosphit Diethylmaleat | 133 | > 6 Tage |
| 9 | 5 | Tris(2,4-di-tert-butylphenyl)phosphit Diethylmaleat | 118 | > 6 Tage |
| 10 | 6 | 1-Ethinyl-1-cyclohexanol | 159 | > 6 Tage |
| 11 | 7 | 1-Ethinyl-1-cyclohexanol | 124 | 5 Tage |

## Patentansprüche

1. Platinkatalysator (D) der allgemeinen Formel (I),
R¹₂Pt[P(OR²)₃]₂ (I)
wobei
R¹ unbahängig voneinander, gleich oder verschieden, ein
- Halogen,
- einfach negativer anorganischer Rest,
- lineare oder verzweigte aliphatische Reste der allgemeinen Formeln CᵥH₂ᵥ₊₁ oder CᵥH₂ᵥ₋₁ mit v = 1 bis 18, Arylalkylreste mit 6 bis 31 Kohlenstoffatomen
- -O-Alkyl, -O-Aryl, -O-Aryl-Alkyl
- -Si(Alkyl)₃, -Si(Aryl)₃, -Si(Aryl-Alkyl)₃,
R² unabhängig voneinander, gleich oder verschieden, ein
- Arylalkyl der Formel - (C₆H₅₋ₚ) - (CₒH₂ₒ₊₁)ₚ mit o = 1 - 31 und p = 1 - 5, wobei mindestens ein Alkylsubstituent an der 2-Position des Phenylrings gebunden ist,
wobei die Wasserstoffatome der für R¹ und R² oben genannten Verbindungen subtituiert oder nicht substituiert sind durch die Gruppen -NH₂, -COOH, -F, -Br, -C1, -Aryl oder -Alkyl
bedeuten.

2. Verwendung des Platinkatalysators gemäß Anspruch 1 als Katalysator für Hydrosilylierungsreaktionen, für die Hydrierung ungesättigter organischer Verbindungen oder Polymere und zur Oligomerisierung von Alkinen.

3. Additionsvernetzende Siliconzusammensetzungen, ausgewählt aus der Gruppe enthaltend
- jeweils mindestens eine Verbindung (A), (B) und (D),
- jeweils mindestens eine Verbindung (C) und (D), und
- jeweils mindestens eine Verbindung (A), (B), (C) und (D) wobei
(A) eine organische Verbindung oder eine Siliziumorganische Verbindung, enthaltend mindestens zwei Reste mit aliphatischen Kohlenstoff-Kohlenstoff-Mehrfachbindungen,
(B) ein Siliziumorganische Verbindung, enthaltend mindestens zwei Si-gebundenen Wasserstoffatome,
(C) eine Siliziumorganische Verbindung, enthaltend SiC-gebundene Reste mit aliphatischen Kohlenstoff-Kohlenstoff-Mehrfachbindungen und Si- gebundene Wasserstoffatome, und
(D) ein Platinkatalysator
bedeuten,
**dadurch gekennzeichnet, dass** der Platinkatalysator (D) ein Platinkatalysator gemäß Anspruch 1 ist.

4. Additionsvernetzende Siliconzusammensetzungen gemäß Anspruch 3, **dadurch gekennzeichnet, dass** sie als weitere Komponente (E) Inhibitoren oder-Stabilisatoren oder eine Mischung mindestens zweier Komponenten (E) in einem Mengenanteil von 0,00001 % bis 5 % bezogen auf das Gesamtgewicht der Zusammensetzung enthalten.

5. Additionsvernetzende Siliconzusammensetzungen gemäß einem der Ansprüche 3 oder 4, **dadurch gekennzeichnet, dass** sie als weitere Komponente (F) enthalten, ausgewählt aus der Gruppe enthaltend verstärkende und nicht verstärkende Füllstoffe, Dispergierhilfsmittel, Lösungsmittel, Haftvermittler, Pigmente, Farbstoffe, Weichmacher, organische Polymere, Hitzestabilisatoren, Fungizide, Duftstoffe, rheologische Additive, Korrosionsinhibitoren, Oxidationsinhibitoren, Lichtschutzmittel, flammabweisend machende Mittel und Mittel zur Beeinflussung der elektrischen Eigenschaften.

6. Additionsvernetzende Siliconzusammensetzungen gemäß einem der Ansprüche 3 bis 5, **dadurch gekennzeichnet, dass** sie als weiteren Bestandteil mindestens einen additionsvernetzenden Katalysatoren (G) enthalten.

7. Verfahren zur Herstellung einer additionsvernetzenden Siliconzusammensetzungen gemäß einem der Anspruch 3 bis 6, **dadurch gekennzeichnet, dass**
- jeweils mindestens eine Verbindung (A), (B) und (D),
- jeweils mindestens eine Verbindung (C) und (D), oder
- jeweils mindestens eine Verbindung (A), (B), (C) und (D) miteinander vermischt werden.

8. Verfahren gemäß Anspruch 7, **dadurch gekennzeichnet, dass** jeweils zusätzlich noch weitere Verbindungen aus der Gruppe der Verbindungen enthaltend (E), (F), (G) eingemischt werden.

9. Verwendung der Zusammensetzung gemäß einem der Ansprüche 3 bis 6 zur Herstellung von Formkörpern, für Siliconbeschichtung, für Imprägnierung, zur Herstellung von Abformungen, für Dicht- Einbett- und Vergussanwendungen.

## Claims

1. Platinum catalyst (D) of the general formula (I),
R¹₂Pt[P(OR²)₃]₂ (I)
where
R¹ are identical or different and are each, independently of one another,
- a halogen,
- a singularly negatively charged inorganic radical,
- linear or branched aliphatic radicals of the general formula CᵥH₂ᵥ₊₁ or CᵥH₂ᵥ₋₁ where v = 1 to 18, arylalkyl radicals having from 6 to 31 carbon atoms,
- -O-alkyl, -O-aryl, -O-arylalkyl,
- -Si(alkyl)₃, -Si(aryl)₃, -Si(arylalkyl)₃,
the radicals R² are identical or different and are each, independently of one another,
- an arylalkyl radical of the formula - (C₆H₅₋ₚ) - (CₒH₂ₒ₊₁) where o = 1 - 31 and p = 1 - 5, where at least one alkyl substituent is present in the 2 position of the phenyl ring,
where the hydrogen atoms of the compounds mentioned above for R¹ and R² are unsubstituted or substituted by the groups -NH₂, -COOH, -F, -Br, -Cl, -aryl or -alkyl.

2. Use of the platinum catalyst according to Claim 1 as catalyst for hydrosilylation reactions, for the hydrogenation of unsaturated organic compounds or polymers and for the oligomerization of alkynes.

3. Addition-crosslinking silicone compositions selected from the group consisting of
- in each case at least one compound (A), (B) and (D),
- in each case at least one compound (C) and (D), and
- in each case at least one compound (A), (B), (C) and (D)
where
(A) is an organic compound or an organosilicon compound comprising at least two radicals having aliphatic carbon-carbon multiple bonds,
(B) is an organosilicon compound containing at least two Si-bonded hydrogen atoms,
(C) is an organosilicon compound containing SiC-bonded radicals having aliphatic carbon-carbon multiple bonds and Si-bonded hydrogen atoms, and
(D) is a platinum catalyst,
**characterized in that** the platinum catalyst (D) is a platinum catalyst according to Claim 1.

4. Addition-crosslinking silicone compositions according to Claim 3, **characterized in that** they contain, as further component (E), inhibitors or stabilizers or a mixture of at least two components (E) in a proportion of from 0.00001% to 5% based on the total weight of the composition.

5. Addition-crosslinking silicone compositions according to either of Claims 3 and 4, **characterized in that** they contain further components (F) selected from the group consisting of reinforcing and nonreinforcing fillers, dispersants, solvents, bonding agents, pigments, dyes, plasticizers, organic polymers, heat stabilizers, fungicides, fragrances, rheological additives, corrosion inhibitors, oxidation inhibitors, light stabilizers, flame retardants and agents for influencing the electrical properties.

6. Addition-crosslinking silicone compositions according to any of Claims 3 to 5, **characterized in that** they contain at least one addition-crosslinking catalyst (G) as further constituent.

7. Process for producing addition-crosslinking silicone compositions according to any of Claims 3 to 6, **characterized in that**
- in each case at least one compound (A), (B) and (D),
- in each case at least one compound (C) and (D), or
- in each case at least one compound (A), (B), (C) and (D)
are mixed with one another.

8. Process according to Claim 7, **characterized in that** further compounds from the group of compounds consisting of (E), (F), (G) are additionally mixed in.

9. Use of the composition according to any of Claims 3 to 6 for producing shaped bodies, for silicone coating, for impregnation, for producing impressions, for sealing, embedding and potting applications.

## Revendications

1. Catalyseur de platine (D) de formule générale (I)
R¹₂Pt[P(OR²)₃]₂ (I)
où
R¹ sont identiques ou différents et signifient, indépendamment l'un de l'autre
- un halogène,
- un radical inorganique portant une seule charge négative,
- des radicaux aliphatiques linéaires ou ramifiés des formules générales CᵥH₂ᵥ₊₁ ou CᵥH₂ᵥ₋₁, v = 1 à 18, des radicaux arylalkyle comprenant 6 à 31 atomes de carbone
- -O-alkyle, -O-aryle, -O-arylalkyle
- -Si(alkyle)₃, -Si(aryle)₃, -Si(arylalkyle)₃,
R² sont identiques ou différents et signifient, indépendamment l'un de l'autre
- un arylalkyle de formule - -(C₆H₅₋ₚ)-(CₒH₂₀₊₁)ₚ, o = 1-31 et p = 1-5, au moins un substituant alkyle étant lié en 2ème position du cycle phényle,
les atomes d'hydrogène des composés susmentionnés pour R¹ et R² étant remplacés ou non remplacés par les groupes -NH₂, -COOH, -F, -Br, -Cl, -aryle ou -alkyle.

2. Utilisation du catalyseur de platine selon la revendication 1 comme catalyseur pour des réactions d'hydrosilylation, pour l'hydrogénation de composés ou de polymères organiques insaturés ou pour l'oligomérisation d'alcynes.

3. Compositions de silicone réticulant par addition, choisies dans le groupe contenant
- à chaque fois au moins un composé (A), (B) et (D),
- à chaque fois au moins un composé (C) et (D) et
- à chaque fois au moins un composé (A), (B), (C) et (D),
(A) signifiant un composé organique ou un composé organosilicié, contenant au moins deux radicaux présentant des liaisons carbone-carbone aliphatiques multiples,
(B) signifiant un composé organosilicié, contenant au moins deux atomes d'hydrogène liés par Si,
(C) signifiant un composé organosilicié, contenant des radicaux liés par SiC, présentant des liaisons carbone-carbone aliphatiques multiples et des atomes d'hydrogène liés par Si, et
(D) signifiant un catalyseur de platine **caractérisées en ce que** le catalyseur de platine (D) est un catalyseur de platine selon la revendication 1.

4. Compositions de silicone réticulant par addition selon la revendication 3, **caractérisées en ce qu'**elles contiennent, comme autres composants (E), des inhibiteurs ou des stabilisateurs ou un mélange d'au moins deux composants (E) en une proportion de 0,00001% à 5% par rapport au poids total de la composition.

5. Compositions de silicone réticulant par addition selon l'une quelconque des revendications 3 ou 4, **caractérisées en ce qu'**elles contiennent d'autres composants (F), choisis dans le groupe contenant les charges renforçantes et non renforçantes, les adjuvants de dispersion, les solvants, les promoteurs d'adhérence, les pigments, les colorants, les plastifiants, les polymères organiques, les stabilisateurs à la chaleur, les fongicides, les parfums, les additifs rhéologiques, les inhibiteurs de corrosion, les inhibiteurs d'oxydation, les agents de protection contre la lumière, les agents ignifuges et les agents pour influencer les propriétés électriques.

6. Compositions de silicone réticulant par addition selon l'une quelconque des revendications 3 à 5, **caractérisées en ce qu'**elles contiennent comme autre constituant au moins un catalyseur réticulant par addition (G).

7. Procédé pour la préparation de compositions de silicone réticulant par addition selon l'une quelconque des revendications 3 à 6, **caractérisé en ce qu'**on mélange les uns avec les autres
- à chaque fois au moins un composé (A), (B) et (D),
- à chaque fois au moins un composé (C) et (D) ou
- à chaque fois au moins un composé (A), (B), (C) et (D).

8. Procédé selon la revendication 7, **caractérisé en ce qu'**on mélange à chaque fois en plus encore d'autres composés du groupe des composés contenant (E), (F), (G).

9. Utilisation de la composition selon l'une quelconque des revendications 3 à 6 pour la fabrication de corps moulés, pour le revêtement au silicone, pour l'imprégnation, pour la fabrication de moulages, pour des applications d'étanchéité, d'enrobage et de coulage.
